# EUROPEAN PATENT SPECIFICATION

(11) **EP 1 115 416 B1**
(45) Date of publication and mention of the grant of the patent: **09.01.2008**
(21) Application number: 99952872.2
(22) Date of filing: 27.09.1999
(51) Int. Cl.: A61K 38/11, A61P 25/24

(54) **USE OF SUBSTANCES WITH OXYTOCIN ACTIVITY IN ORDER TO CREATE CELL REGENERATION**
VERWENDUNG VON SUBSTANZEN MIT OXYTOCIN AKTIVITÄT MIT DEM ZIEL DER ZELLREGENERATION
UTILISATION DE SUBSTANCES AYANT UNE ACTIVITE OXYTOCIQUE POUR LA REGENERATION CELLULAIRE

(30) Priority: 25.09.1998 SE 9803272
(43) Date of publication of application: 18.07.2001
(73) Proprietor: EntreTech Medical AB, 182 62 Djursholm (SE)
(72) Inventor: UVNÄS-MOBERG, Kerstin, S-182 62 Djursholm (SE); LUNDEBERG, Thomas, S-181 31 Lidingö (SE)
(74) Representative: Skoglösa, Ylva
(86) International application number: PCT/SE1999/001713
(87) International publication number: WO 2000/018424

(56) References cited:
- WO-A1-98/43661
- DE-A- 4 236 293
- DE-A1- 4 312 913
- KERSTIN UVNCS-MOBERG: 'Oxytocin May Mediate the Benefits of Positive Social Interaction and Emotions' PSYCHONEUROENDOCRINOLOGY vol. 23, no. 8, November 1998, SWEDEN, pages 819 - 835, XP002921910
- ANTONIO ARGIOLAS ET AL: 'Central Functions of Oxytocin' NEUROSCIENCE & BIOBEHAVIORAL REVIEWS vol. 15, 1991, USA, pages 217 - 231, XP002921914
- STN International, File CAPLUS, CAPLUS accession no. 1995:339170, Document no. 122:96599, Levay, P.F. et al: "Oxytocin: a Short Review", S.-Afr. Tydskr. Natuurwet. Tegnol. (1993), 12(3), XP002940196
- ROBINSON I.C.A.F.: 'The development and evaluation of a sensitive and specific radioimmunoassay for oxytocin in unextracted plasma' JOURNAL OF IMMUNOASSAY vol. 1, no. 3, 1980, MARCEL DEKKER, BASEL, CH, pages 323 - 347, XP009018099

## Description

The present invention relates to the use of substances with oxytocin activity for the preparation of a pharmaceutical in order to create cell regeneration in certain diseases. It also relates to a cosmetic method for treatment of aged skin and stimulate the growth of hair and nails, wherein a substance with oxytocin activity is administrated to an individual.

### Background of the invention

Oxytocin was one of the first peptide hormones to be isolated and sequenced. It is a nonapeptide with two cysteine residues that form a disulfide bridge between positions 1 and 6 and corresponds to the formula

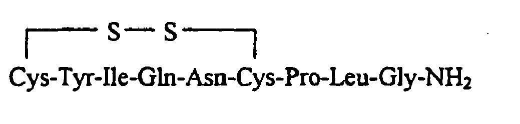

For a long time the only effects attributed to oxytocin were its stimulating effects on milk ejection and uterine contractions, but in the past decades it has been shown that oxytocin exerts a wide spectrum of effects within the central nervous system, CNS. It has been suggested that oxytocin participates in the control of memory and learning processes and of various types of behaviour such as feeding, locomotion, as well as maternal and sexual behaviour. Oxytocin is also suggested to participate in the control of cardiovascular functions, thermoregulation, pain threshold and fluid balance. There is also evidence that oxytocin is involved in the control of various immunological processes. It has recently been demonstrated that oxytocin injections cause a lowering of blood pressure and increased weight gain - long lasting effects after repetitive administration. As a central stimulating substance oxytocin plays an important role in the interaction between mother and progeny in mammals. The products may also be used prophylactic in young human beings e.g. already in new born babies or young children to prevent the development of diseases later on in life which diseases are dependent on stress conditions during the fetal life. Such conditions may be heart/vessel diseases such as stroke, heart infarct, hypertension, and diabetes.

There are different processes described for the synthetical production of oxytocin; commercial processes are for instance described in US patents 2,938,891 and 3,076,797.

In the human body oxytocin is produced in the paraventricular nucleus, PVN, and the supraoptic nucleus, SON, of the hypothalamus. It differs by only two amino acids from vasopressin, which is also produced in these nuclei. The magnocellular oxytocinergic neurons of the SON and PVN send Parvocellular neurons that originate in the PVN project into multiple areas within CNS. The oxytocin-producing cells are innervated by cholinergic, catecholaminergic as well as peptidergic neurons. The presence of oxytocin in different tissues outside the brain, such as the uterus, ovaries, testis, thymus, adrenal medulla and pancreas has been demonstrated and oxytocin is suggested to exert local effects in these organs.

A parallel secretion of oxytocin into the brain regions and into the circulation occurs in response to some stimuli such as suckling, but other stimuli can cause separate activation of oxytocinergic neurons, terminating in the brain or the pituitary.

In this context oxytocin refers, whenever applicable, in addition to oxytocin also to precursors, metabolic derivatives, oxytocin agonists or analogues displaying the same properties.

There are several oxytocin derivatives, i.e. compounds with a structure similar to that of oxytocin. There are preliminary indications that other oxytocin derivatives than oxytocin could give the cell regeneration effects of oxytocin as well as parts of the oxytocin molecule. No publications describe the use of other oxytocin derivatives than oxytocin or parts of the oxytocin molecule to improve cell regeneration.

In experiments it has been shown that oxytocin by way of a central action increases the activity of the central α₂-receptors in rats. These receptors have an inhibitory action and counteracts the activating aspects of noradrenalin in the brain which are mainly mediated via α₁-receptors, which activate cyclic AMP. When α₂-receptor stimulation dominates over α₁-receptor stimulation, activity is exchanged by relaxation and energy is shunted towards growing and healing, i.e. is not used for stress or muscular contraction and activity. As a consequence parasympathetic nerve tone dominates over sympathetic nervous tone and the musculature is relaxed. It can be presumed that oxytocin exerts a similar effect also in humans. During breast feeding - a situation characterized by repetitive oxytocin secretion - all the effects observed in experimental animals following repeated oxytocin administration are seen. It is not known how the effect by oxytocin on α₂-receptors is mediated, but probably not by a classical oxytocin receptor mediated effect.

The effect of oxytocin can be extended or strengthened by administration in combination with drugs increasing the release of oxytocin and/or the number or affinity of receptors, such as estrogen, or drugs having an α₂-agonistic effect, such as clonidine.

It has now turned out that oxytocin stimulates fetal growth (Example 1), stimulates recovery of peripheral and central neuropathies (Example 2), stimulates recovery of peripheral and central neuropathies (Example 2), stimulates human dermal dendritic cells (Example 3), stimulates dermal keratinocytes (Example 4), reduces apoptosis (Example 5), has effects on aged skin and barrier formation (Example 6). These Examples indicate that oxytocin or that substances with oxytocin activity may be used for cell regeneration.

### Summary of the invention

The present invention relates to the use o a substance with oxytocin activity for the preparation of a pharmaceutical in order to improve cell regeneration in certain diseases according to claim 1. The invention also relates to a cosmetic method for treatment of aged skin and stimulation of the growth of hair and nails, wherein a substance with oxytocin activity is administrated to an individual.

### Detailed description of the invention

An object of the present invention is the use of a substance with an oxytocin like activity in order to create cell regeneration. Examples of substances with oxytocin activity are the following compounds: 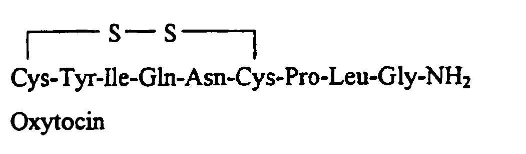 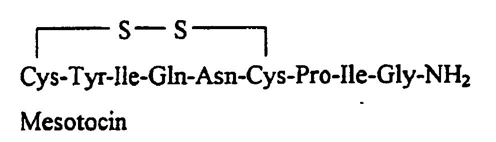 i.e. V is Tyr, W is Ile, X is GIn, Y is Ile, and Z is Gly in Claim 2 and 4 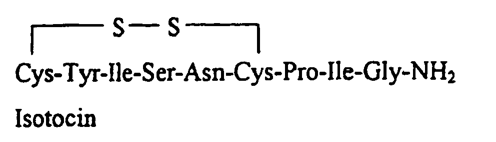 i.e. V is Tyr, W is Ile, X is Ser, Y is Ile, and Z is Gly in Claim 2 and 4 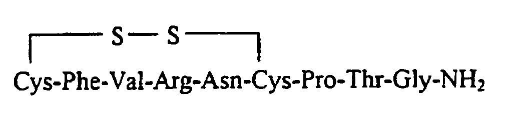 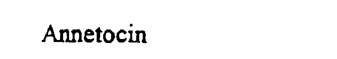 i.e. V is Phe, W is Val, X is Arg, Y is Thr, and Z is Gly in Claim 2 and 4

Annetocin has been isolated from the earthworm, as described in Oumi T, Ukena K, Matsushima O, Ikeda T, Fujita T, Minakata H, Nomoto K, Annetocin: an oxytocin-related peptide isolated from the earthworm, Eisenia foetida, Biochem Biophys Res Commun 1994, Jan 14; 198(1): 393-399. Other substances with oxytocin activity could also be used, such as naturally occurring or artificially modified variants, analogues, and derivatives of oxytocin, mesotocin, isotocin, and annetocin. Such substances could be obtained by addition, insertion, elimination, or substitution of at least one amino acid in these hormones. By substance with an oxytocin like activity is also understood precursors, metabolites such as metabolic derivatives e.g. metabolic degradation products, agonists, or analogues of the substances mentioned herein displaying the same properties. Metabolic derivatives or metabolic degradation products may be oxytocin like peptides e.g. with nine amino acids such as oxytocin, mesotocin, isotocin, and annetocin from which one or more amino acids has been deleted from either or both ends of the molecule. Preferably one, two or three amino acids may have been deleted from the carboxy terminal end i.e. Gly only, Gly and Leu, or Gly, Leu, and Pro. Preferably one, two or three amino acids may have been deleted from the amino terminal end i.e. Cys only, Cys and Tyr, or Cys, Tyr, and Ile. Preferably one, two or three amino acids may have been deleted both from the carboxy terminal end i.e. Gly only, Gly and Leu, or Gly, Leu, and Pro, and one, two or three amino acids may have been deleted from the amino terminal end i.e. Cys only, Cys and Tyr, or Cys, Tyr, and Ile. It could be ascertained that these variants are analogues of oxytocin, mesotocin, isotocin or annetocin by immunological methods, e.g. RIA (radio-immunoassay), IRMA (radiometric methods), RIST (radioimmunosorbent test), RAST (radioallergosorbent test).

As mentioned above there are indications that subfragments of the oxytocin molecule could give cell regeneration. Preferably, these subfragments of the oxytocin molecule is made by deletions of amino acids outside the disulfide bridge. Examples of subfragments of the oxytocin molecule are the following compounds: 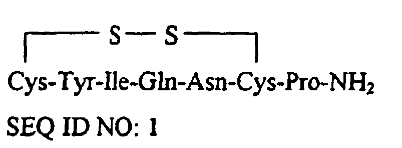 i.e. V is Tyr, W is Ile, X is Gln, Y is nothing, and Z is nothing in Claim 2 and 4 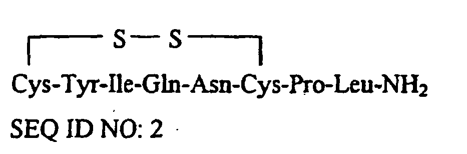 i.e. V is Tyr, W is Ile, X is Gln, Y is Leu, and Z is nothing in Claim 2 and 4

There is also a possibility to create new compounds with oxytocin activity by means of computer simulation. Methods for computer simulation are known by a person skilled in the art, e.g. as described in EP 0660 210 A2. Seven new compounds have been created by means of computer simulation, namely the following peptides: 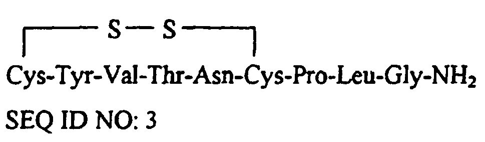 i.e. V is Tyr, W is Val, X is Thr, Y is Leu, and Z is Gly in Claim 2 and 4 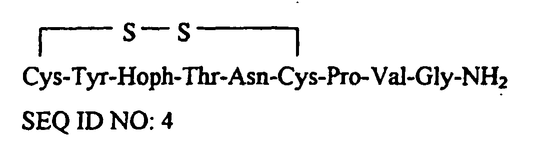 i.e. V is Tyr, W is Hoph, X is Thr, Y is Val, and Z is Gly in Claim 2 and 4 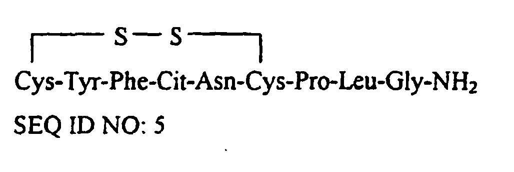 i.e. V is Tyr, W is Phe, X is Cit, Y is Leu, and Z is Gly in Claim 2 and 4 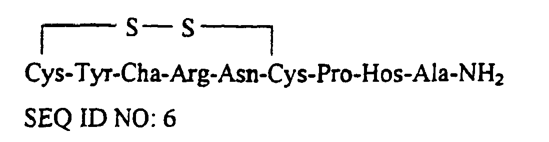 i.e. V is Tyr, W is Cha, X is Arg, Y is Hos, and Z is Ala in Claim 2 and 4 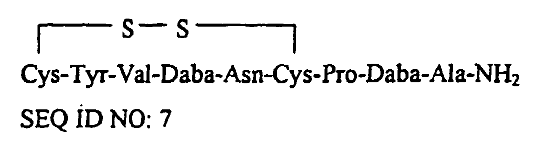 i.e. V is Tyr, W is Val, X is Daba, Y is Daba, and Z is Ala in Claim 2 and 4 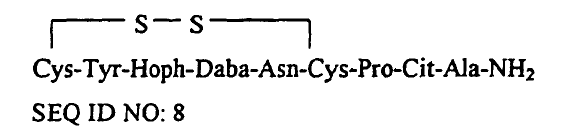 i.e. V is Tyr, W is Hoph, X is Daba, Y is Cit, and Z is Ala in Claim 2 and 4 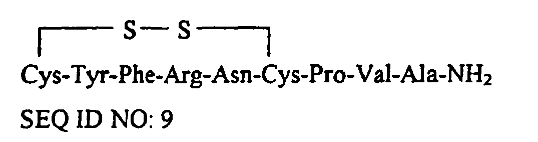 i.e. V is Tyr, W is Phe, X is Arg, Y is Val, and Z is Ala in Claim 2 and 4,
wherein Cha stands for cyclohexylalanine,

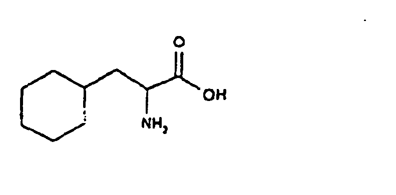

Hoph stands for homophenylalanine,

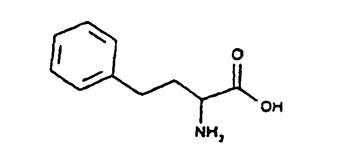

Cit stands for citruline,

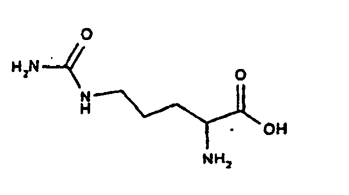

Daba stands for diaminobutyric acid, and

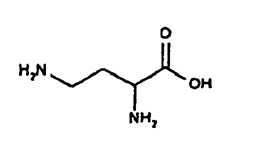

Hos stands for homoserine.

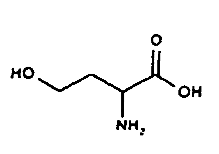

The invention also relates to the use of the peptides mentioned above in both D- and L-form. Especially the invention relates to the use of the L-form. By inversion of the peptide sequence thereof, the D-form could be converted to the L-form. The effect of the D- and L-forms are the same. These and the peptides above can be produced by methods known to a person skilled in the art, e.g. according to Merrifield, P.B., "Solid Phase Synthesis", Angew. Chemie, 1985, No. 97, p. 801.

It could be ascertained that compounds have oxytocin effect by tests, such as described in the publication by Cassoni, P. below and the tests according to the Examples that follow.

The improvement of cell regeneration according to the invention relates to conditions chosen from Parkinson's disease, after transplantations, to recover proliferation of blood cells improve damaged sight, hearing, olfactory sense, and taste as a result of aging, and to relieve incontinence problems. The invention also relates to a cosmetic method in order to rejuvenate skin and counteract wrinkles as a result of aging, preoperatively to increase the healing capacity, and to assist in the growth of hair and nails, according to claim 7.

A pharmaceutical composition to be used according to the invention may comprise an effective concentration of at least one substance with oxytocin activity in mixture or otherwise together with at least one pharmaceutically acceptable carrier or excipient.

Examples of substances with oxytocin activity are mesotocin, isotocin, annetocin. SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, and SEQ ID NO: 9.

The pharmaceutical compositions to be used according to the invention may contain substances that extend or strengthen the effects of oxytocin. Such substances could increase the release of oxytocin and/or the number or affinity of receptors, such as estrogen, or drugs having an α₂-agonistic effect, such as clonidine.

The pharmaceutical compositions are prepared in a manner known to a person skilled in the pharmaceutical art. The carrier or the excipient could be a solid, semi-solid or liquid material that could serve as a vehicle or medium for the active ingredient. Suitable carriers or excipients are known in the art. The pharmaceutical composition could be adapted to oral, parenteral, or topical use and could be administered to the patient as tablets, capsules, suppositories, solutions, suspensions or the like.

The pharmaceutical compositions could be administered orally, e.g. with an inert diluent or with an edible carrier. They could be enclosed in gelatin capsules or be compressed to tablets. For oral therapeutic administration the compounds according to the invention could be incorporated with excipients and used as tablets, lozenges, capsules, elixirs, suspensions, syrups, wafers, chewing gums and the like. These preparations should contain at least 4% by weight of the compounds according to the invention, the active ingredient, but could be varied according to the special form and could, suitably, be 4-70% by weight of the unit. The amount of the active ingredient that is contained in compositions is so high that a unit dosage form suitable for administration is obtained.

The tablets, pills, capsules, lozenges and the like could also contain at least one of the following adjuvants: binders such as microcrystalline cellulose, gum tragacanth or gelatin, excipients such as starch or lactose, disintegrating agents such as alginic acid, Primogel, com starch, and the like, lubricants such as magnesium stearate or Sterotex, glidants such as colloidal silica dioxide, and sweetening agents such as saccharose or saccharin could be added or flavourings such as peppermint, methyl salicylate or orange flavouring. When the unit dosage form is a capsule it could contain in addition of the type above a liquid carrier such as polyethylene glycol or a fatty oil. Other unit dosage forms could contain other different materials that modify the physical form of the unit dosage form, e.g. as coatings. Accordingly, tablets or pills could be coated with sugar, shellac or other enteric coating agents. A syrup could in addition to the active ingredient contain saccharose as a sweetening agent and some preservatives, dyes and flavouring agents. Materials that are used for preparation of these different compositions should be pharmaceutically pure and nontoxic in the amounts used.

For parenteral administration the compounds could be incorporated in a solution or suspension. Parenteral administration refers to the administration not through the alimentary canal but rather by injection through some other rute, as subcutaneous, intramuscular, intraorbital, intracapsular, intraspinal, intrasternal, intravenous, intranasal, intrapulmonary, through the urinary tract, through eye drops, rectal or vaginal (e.g. as a suppository, a vagitorium, a cream or an ointment), through the lactiferous tract in cattles, into an organ such as bone marrow, etc. Bone marrow may also be treated *in vitro.* These preparations could contain at least 0.1% by weight of an active compound according to the invention but could be varied to be approximately 0.1-50% thereof by weight. The amount of the active ingredient that is contained in such compositions is so high that a suitable dosage is obtained.

The solutions or suspensions could also comprise at least one of the following adjuvants: sterile diluents such as water for injection, saline, fixed oils, polyethylene glycols, glycerol, propylene glycol or other synthetic solvents, antibacterial agents such as benzyl alcohol or methyl paraben, antioxidants such as ascorbic acid or sodium bisulfite, chelating agents such as ethylene diamine tetraacetic acid, buffers such as acetates, citrates or phosphates, and agents for adjustment of the tonicity such as sodium chloride or dextrose. The parenteral preparation could be enclosed in ampoules, disposable syringes or multiple dosage vessels made of glass or plastic.

For topical administration the compounds according to the invention could be incorporated in a solution, suspension, or ointment. These preparations could contain at least 0.1% by weight of an active compound according to the invention but could be varied to be approximately 0.1-50% thereof by weight. The amount of the active ingredient that is contained in such compositions is so high that a suitable dosage is obtained. The administration could be facilitated by applying touch, pressure, massage, heat, warms, or infrared light on the skin, which leads to enhanced skin permeability. Hirvonen, J., Kalia, YN, and Guy, RH. Transdermal delivery of peptides by iontophoresis, Nat Biotechnol 1996 Dec; 14(13): 1710-1713 describes how to enhance the transport of a drug via the skin using the driving force of an applied electric field. Preferably, iontophoresis is effected at a slightly basic pH.

Other administration forms are inhalation through the lungs, buccal administration via the mouth and enteral administration via the small intestine that could be effected by means known by a person skilled in the art.

By the expression "comprising" we understand including but not limited to. Thus, other non-mentioned substances, additives or carriers may be present.

### Short description of the Figures

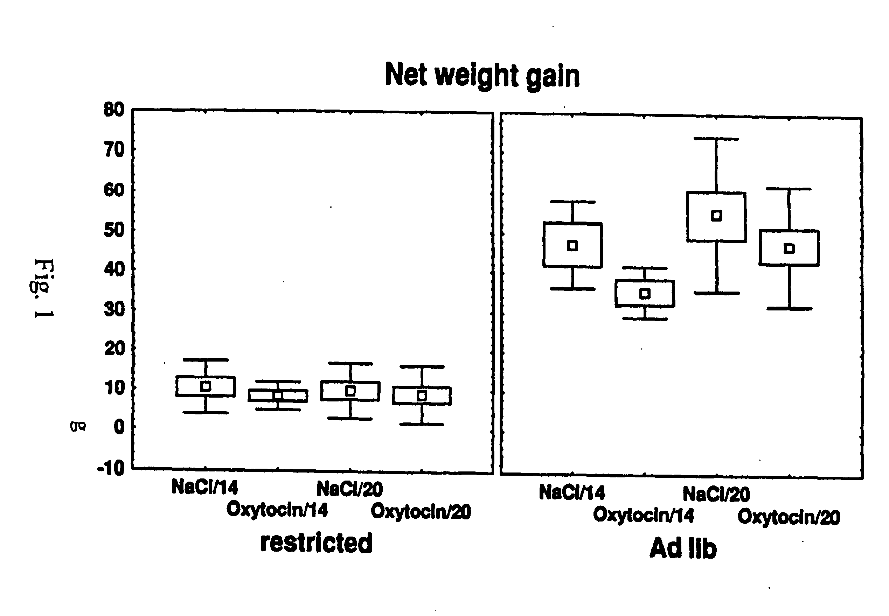

**Figure 1**. Maternal net weight (total body weight - weight of the uterus including fetal and placental tissues) gain during the first 14 and 20 days of gestation, in food restricted and ad libitum fed, NaCl- and oxytocin-treated dams. Effect of nutrition (p<0.001), effect of treatment (p=0.06), effect of day of gestation (ns), no significant interactions. The boxes indicate SEM and the bars SD.

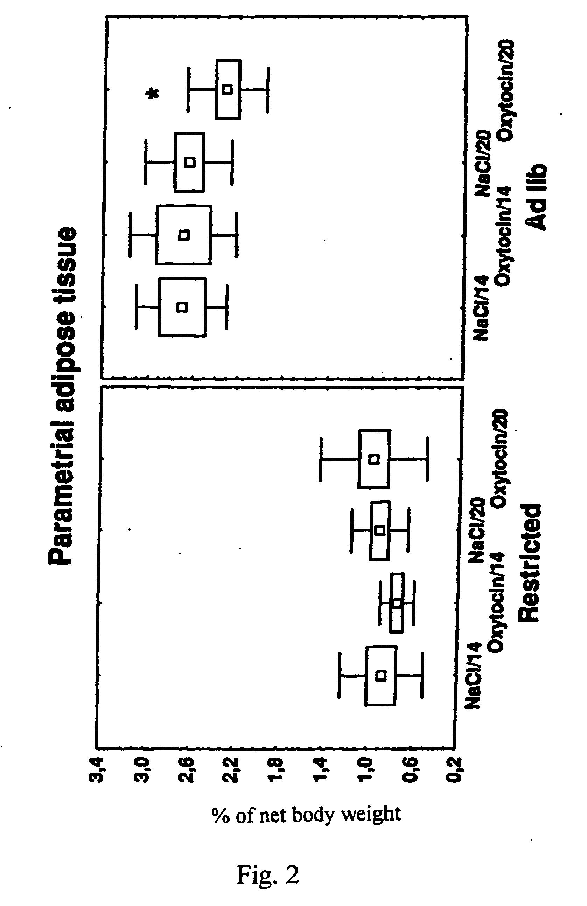

**Figure 2.** Parametrial adipose tissue, on gestational days 14 and 20, in food restricted and ad libitum fed, NaCl- and oxytocin-treated dam. Effect of nutrition (p<0.001), effect of treatment (ns), effect of day of gestation (ns), interaction (day of gestation x treatment) (p<0.05), no other significant interactions. *Significant (p<0.05) difference between ad libitum fed oxytocin- and NaCl-treated groups on gestational day 20. The boxes indicate SEM and the bars SD.

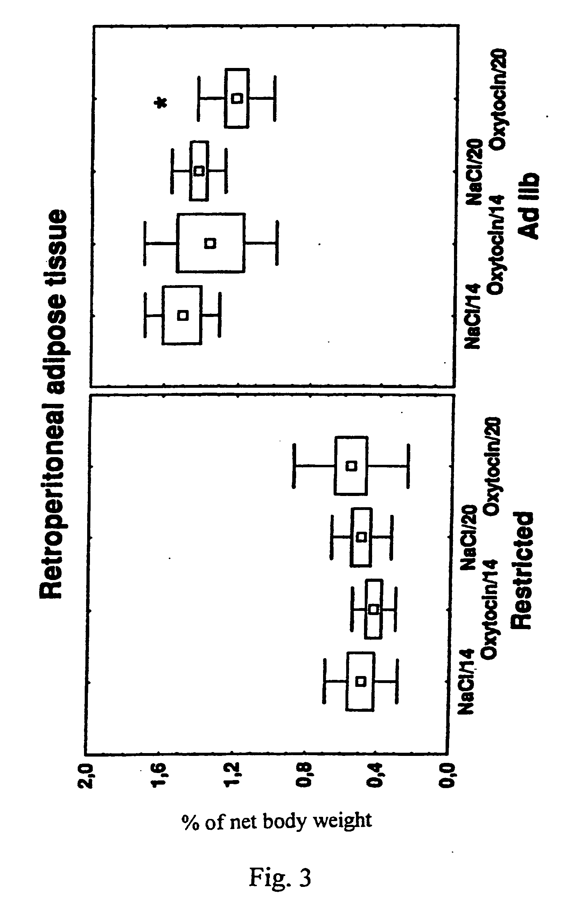

**Figure 3.** Retroperitoneal adipose tissue, on gestational days 14 and 20, in food restricted and ad libitum fed, NaCl- and oxytocin-treated dams. Effect of nutrition (p<0.001), effect of treatment (ns), effect of day of gestation (ns), interaction (day of gestation x treatment) (p<0.05), no other significant interactions. *Significant (p<0.05) difference between ad libitum fed oxytocin- and NaCl-treated groups on gestational day 20. The boxes indicated SEM and the bars SD.

The invention will be illuminated by the following Examples, which are only intended to illuminate and not restrict the invention in any way.

### Examples

### Example 1 - Stimulation of fetal growth

### Materials and methods

### Animals

Nullipara female Sprague-Dawley rats (body weight ± 12 g) were obtained from B&K Universal AB, Sollentuna, Sweden. Animals were single housed in cages in temperature (20 ± 1°C)- and humidity (45-55%)-controlled rooms, illuminated from 0600 h to 1800 h. The diet was a pelleted ration (Lactamin, Vadstena, Sweden). The animals had free access to tap water. Body weight of dams were monitored weekly during the first four weeks and on gestational days 1, 14 and 20 and on lactational day 1 and 20. Body weight of pups were recorded on lactational days 1, 5, 10, 15 and 20. The study was approved by the Stockholm Ethical Committee for Experiments in Animals.

### Animal experiments

At arrival, animals were divided into two groups, one ad libitum fed and one food restricted. The food restricted animals obtained 70% of the average food consumed by the ad libitum fed animals throughout the experiment. After four weeks, the rats were mated and considered to be pregnant (gestational day 1); the morning sperm was found in their vaginal smear. Ad libitum fed and food restricted pregnant rats were divided into two subgroups, oxytocin treated and control. Oxytocin-treated animals were injected once a day with oxytocin (1 mg/kg body weight) while control animals were injected with saline (NaCl 0.9%) subcutaneously, during gestational day 1-5. Ad libitum fed and food restricted animals were killed on gestational days 14 and 20, and groups of ad libitum fed dams and pups were killed on lactational day 20. Pregnant rats delivered their pups late in the afternoon on gestational day 23. The day after was considered as lactational day 1. On that day, the litter was culled to 8 pups/dam. Virginal, ad libitum fed and food restricted, rats were killed at the time corresponding to day 1 of gestation for the mated rats. The following 12 groups were thus included in the experiment: NaCl-treated, virginal, ad libitum fed (n=8), NaCl-treated, virginal, food restricted (n=8), NaCl-treated, gestational day 14, ad libitum fed (n=4), NaCl-treated, gestational day 14, food restricted (n=8), NaCl-treated, gestational day 20, ad libitum fed (n=10), NaCl-treated, gestational day 20, food restricted (n=10), NaCl-treated, lactational day 20, ad libitum fed (n=4), oxytocin-treated, gestational day 14, ad libtum fed (n=4), oxytocin-treated, gestational day 14, food restricted (n=9), oxytocin-treated, gestational day 20, ad libtium fed (n=12), oxytocin-treated, gestational day 20, food restricted (n=13), oxytocin-treated, lactational day 20, ad libitum fed (n=3).

Maternal trunk blood was collected by decapitation. Maternal adipose tissue from the parametrial and retroperitoneal region, the liver, individual fetuses and placentas (only gestational day 20), were dissected out and weighed. Immediately after decapitation, trunk blood was collected in chilled plastic tubes containing 10 IU/mL heparin (Lövens Läkemedel, Malmö, Sweden) and 500 IU/mL aprotinin (Bayer AB, Stockholm, Sweden). The samples were centrifuged immediately, and the plasma was harvested and frozen (-20°C).

### Analysis of blood samples

Oxytocin was determined with radioimmunoassay (RIA), after purification of plasma (500 µl) on Sep-Pak C18 cartridges (Water Corporation, Milford, MA, USA) as described by Marchini G, Lagercrantz H, Uvnäs-Moberg K. Plasma gastrin i newborn infants and their relationship tp catecholamines. J Dev Physiol 1990, 14:147-155, and dissolved in 500 µl assay buffer (0.05 M phosphate buffert with 0.1% BSA, pH 7.5). The concentration of oxytocin was determined in the purified plasma (100 µl) incubated with 50 µl of the antibody A19 (Euro-Diagnostica AB, Malmö, Sweden) at +4°C for 24h. 50 µl of tracer (¹²⁵I)-Tyr²-oxytocin, specific activity 2200 Ci/mmol (DuPont NEN Research Products, Boston, MA, USA) was added and the incubation continued at +4°C for 48 h. Antibody and (¹²⁵I)-Tyr²-oxytocin were diluted in 0.05 M phosphate buffer with 0.1% BSA. Cross-reactivity with arginine-vasotocin was 0.01%, with lysine-vasopressin <0.01% and with arginine-vasotocin 0.1%. The bound fraction was separated from the unbound by incubation of the samples with a second rabbit antibody Decanting Suspension 3 (Pharmacia & Up-john Diagnostics AB, Uppsala, Sweden). The samples were centrifuged (1700 x g) for 10 min, the supernatant decanted and the radioactivity in the precipitate determined. The limit of detection was 3.2 pmol/l. The intra- and inter-assay coefficients of variation were 18% and 26%, respectively.

### Statistical analysis

Results are presented as mean ± SD. The effect of pregnancy in ad libitum fed and food restricted control animals was assessed by two-way ANOVA, having nutrition (ad libitum fed vs food restricted) and day of gestation (virginal, day 14 and day 20) as components of variation. The effect of oxytocin-treatment was assessed by a three-way ANOVA, having nutrition (ad libitum fed and food restricted), treatment (oxytocin vs saline) and day of gestation (day 14 vs day 20) as components of variation. If a significant interaction was found, planned comparisons were used to assess differences between oxytocin and corresponding NaCl treated group. Differences in proteolytic activity and differences in postnatal pup weight, between oxytocin and NaCl treated groups, were assessed by Student's t-test for independent means. Results were considered significant when p<0.05.

### Results

Oxytocin-treated rats tended (p=0.06) to have a smaller net weight gain during gestation than their NaCl-treated counterparts (Figure 1). On gestational day 20, ad libitum fed oxytocin-treated rats contained relatively (% of net body weight) less parametrial (Figure 2) and retroperitoneal (Figure 3) adipose tissue compared with saline treated rats, while earlier in pregnancy, there was no difference in the amount of parametrial and retroperitoneal adipose tissue between oxytocin and saline treated rats.

Fetuses and placentas from ad libitum fed oxytocin treated dams were larger on gestational day 20 and the birthweight was higher, compared with conceptus and offspring from NaCl-treated rats (Table 1). No further changes in postnatal body weights between pups from ad libitum fed oxytocin and NaCl-treated dams was observed. In contrast to the ad libitum fed group, there was no effect of oxytocin on fetal weight in the food restricted group (Table 1).

**Table 1. Reproductive outcome of ad libitum fed and food restricted animals. Values are mean ± SD.**

| | **Ad libitum fed** | | **Food restricted** | |
|---|---|---|---|---|
| | **NaCl** | **Oxytocin** | **NaCl** | **Oxytocin** |
| ***Fetal*/*pup weight (g)*** | | | | |
| Gestational day 14^{a} | 0.067 ± 0.011 | 0.069 ± 0.009 | 0.058 ± 0.010 | 0.060 ± 0.014 |
| Gestational day 20^{a} | 2.137 ± 0.168 | 2.275 ± 0.213^{b} | 1.914 ± 0.274 | 1.879 ± 0.255 |
| Lactational day 1 | 7.291 ± 0.232 | 7.619 ± 0.473^{b} | | |
| Lactational day 5 | 14.425 ± 0,512 | 14.783 ± 0.958 | | |
| Lactational day 10 | 26.391 ± 1.285 | 26.648 ± 1.565 | | |
| Lactational day 15 | 39.350 ± 2.315 | 40.417± 2.489 | | |
| Lactational day 20 | 52.321 ± 3.106 | 54.021 ± 3.253 | | |
| Placental weight (g) | | | | |
| Gestational day 20^{a} | 0.476 ± 0.086 | 0.530 ± 0.089^{b} | 0.429 ± 0.058 | 0.412 ± 0.067 |

| | | | | |
|---|---|---|---|---|
| ^{a}Significant difference between ad libitum fed and food restricted groups ^{b}Significant difference between NaCl and oxytocin treated groups | | | | |

### Discussion

Oxytocin had small but significant beneficial effects on fetal growth in ad libitum fed animals, but no further effect on postnatal growth during the lactational period. It is conceivable that other doses of oxytocin, way or ways of administration could further enhance fetal growth. The effects of oxytocin could probably vary depending on when during gestation it is administered. According to the present invention, oxytocin was injected during the first five days of gestation, a period when the fertilised ovum is not yet implanted. Therefore, the stimulatory effects of oxytocin on fetal growth is likely to be explained by altered maternal adaptions to pregnancy, having positive effects on, for example, placental nutrient transfer capacity or the ability of the mother to mobilise her body stores in late pregnancy.

### Example 2 - Recovery of peripheral and central neuropathies

### Materials and methods

### Animals

This animal model is as described in Petersson M, Alster P, Lundeberg T. and Uvnäs-Moberg K. Oxytocin Causes a Long-Term Decrease of Blood Pressure in Female and Male Rats. Physiology & Behavior, Vol. 60, No. 5, pp. 1311-1315, 1996. Experiments were performed in 2 series. Female and male Sprague-Dawley rats (230-250 g) were used for sc injection and male Sprague-Dawley rats (335-375 g) for intracerebroventricular (icv) injection (B&K Universal AB, Sollentuna, Sweden). The animals arrived at least 3 weeks before experiments and were housed 5 per cage, except animals provided with icv cannulas that were housed individually, with free access to food and water. The light schedule was a 12/12h light/dark cycle. The ambient temperature was 20 ± 2°C. In female rats, the stage of the estrous cycle was determined by microscopic examination of vaginal smears.

### Surgery for icv injections

Following anesthesia with pentobarbitalnatrium (Apoteksbolaget, Sweden), 50 mg/kg injected intraperitoneally (ip), the skull was uncovered and a guide cannula (21 g) was stereotactically fixed to the skull by acrylic dental cement. The coordinates were 1.00 mm posterior and 1.30 mm lateral to the bregma. The guides reached, but did not penetrate, the dura mater, with the needle tip in the lateral ventricle. The animals were allowed 1 week of recovery after the operation. At the end of the experiment, the placement of the guide cannula was checked by injection of 2 µl of Toluidine Blue.

### Experimental design

NaCl, oxytocin 0.01, 0.1, and 1 mg/kg (Ferring, Malmö, Sweden) and NaCl again were given sc for consecutive 5-day periods to male and female rats. Five-day treatment periods were chosen to cover the duration of the estrous cycle in female rats. Drugs were dissolved in physiological saline and injected in a volume of 1 mL/kg. Control animals received NaCl sc (1mL/kg) during the entire treatment period.

In a second series of experiments, oxytocin was given icv (1 µg/kg) in a volume of 5 µl during 5 days to male rats provided with chronic icv cannulas. Control animals received 5 µl saline icv.

### The role of NGF

Nerve growth factor (NGF), a powerful agent for the growth, differentiation and regeneration of lesioned cells of the central and peripheral nervous system, has in recent years been indicated as a potential therapeutic agent capable of reversing the process of cell damage in neurodegenerative events in man. Since NGF does not cress the blood brain barrier and central NGF administration requires invasive surgical procedures, the discovery of substances modulating in vivo NGF synthesis in the brain will be extremely useful for a possible clinical use of NGF. Also, NGF is a target-derived growth factor that plays a crucial role in growth and differentiation of peripheral sensory and sympathetic neurons. NGF has been shown to promote survival not only of surgical and chemical injured peripheral nerve cell damage but also of diabetes neuropathies, leprosy, post-ischemic coronary innervation, and erectile function. As a result of these observations NGF has likely a role in the treatment of peripheral neuropathies. Because numerous cells are able to produce local and circulating NGF a possible strategy is to identify compounds that induce up-regulation of NGF in vivo.

### Central effects

The aim of the present study was to analyse if the content of NGF in the brain of adult rats can be affected by peripheral (subcutaneous, sc) administration of oxytocin. The NGF concentrations in the hypothalamus and hippocampus were analysed by the use of a sensitive immunoenzymatic assay for NGF.

Table 2 shows the results that were obtained regarding brain NGF levels (pg/g tissue) in the hippocampus and hypothalamus of rats injected sc with oxytocin compared to the saline control animals.

**Table 2. Brain NGF levels (pg/g tissue) of rats injected sc with oxytocin.**

| Part of the brain | Treated with saline | Treated with oxytocin |
|---|---|---|
| Hippocampus | 4312 ± 213 | 5674 ± 389 |
| Hypothalamus | 447 ± 66 | 1543 ± 455 |

The results in Table 2 suggest that sc injection of oxytocin can modulate the NGF levels in the brain.

### Peripheral effects

To elucidate the peripheral effects of sc oxytocin, two models of peripheral neuropathies in rats were used: one involving the peripheral sensory nervous system (by the use of capsaicin) and one involving the peripheral sympathetic nervous system (by the use of 6-hydroxydopamine, 6-OHDA). In the present study we have used these animal models to investigate whether exogenous administration of oxytocin influences the changes induced by 6-OHDA on the iris/hind paw and of capsaicin on the hind paw. The Tables below show the results that were obtained regarding peripheral NGF levels (pg/g tissue) in iris (Table 3) and hind paw (Table 4 for 6-OHDA and Table 5 for capsaicin) of rats injected sc with oxytocin compared to the saline control animals.

**Table 3. Peripheral NGF levels (pg/g tissue) of rats injected sc with oxytocin in the iris compared to saline control animals, by the use of 6-OHDA.**

| | NGF pg/g |
|---|---|
| 6-OHDA + saline | 834 ± 115 |
| 6-OHDA + oxytocin | 9844 ± 1206 |

**Table 4. Peripheral NGF levels (pg/g tissue) of rats injected sc with oxytocin in the hind paw to saline control animals, by the use of 6-OHDA.**

| | NGF pg/g |
|---|---|
| 6-OHDA + saline | 756 ± 223 |
| 6-OHDA + oxytocin | 6711 ± 987 |

**Table 5. Peripheral NGF levels (pg/g tissue) of rats injected sc with oxytocin in the hind paw to saline control animals, by the use of capsaicin.**

| | NGF pg/g |
|---|---|
| Capsaicin + saline | 1039 ± 438 |
| Capsaincin + oxytocin | 1432 ± 519 |

To elucidate if the treatment had any physiological significance, the hot plate response was used Table 6 shows to the latency to lick (startle response) for animals treated with capsacin + saline and capsaicin + oxytocin, respectively.

**Table 6. Latency to lick (startle response) (in seconds) for rats treated with oxytocin in the hind paw to saline control animals, by the use of capsaicin.**

| | Latency to lick (sec) |
|---|---|
| Capsaicin + saline | 43 ± 15 |
| Capsaicin + oxytocin | 32 ± 9 |

The results in Table 3-6 show that a repeated administration of oxytocin increases the NGF content in the lesioned tissue and this may have functional implications.

### Example 3 - Stimulation of human dermal dendritic cells

### Materials and methods

The tetanus toxoid (TT) model was used that was described by Demotz et al. J Immunol 143:3881-3886. In the present study we set out to investigate the effects of oxytocin on a tetanus toxoid driven T cell proliferation model (proliferation assay). We used TT as a model antigen because most Swedish patients have a history of vaccination for TT and possess circulating TT specific memory T cells. To test the effects of oxytocin, one group of dendritic cells were preincubated with oxytocin and one with saline. Resting CD4 positive T cells were used as responders. The dendritic cells were pulsed with TT for 2h at 37°C and incubated with 5x100,000 responder cells resulting in a stimulator: responder ratio of 1:1,000, 1:100 and 1:10. Proliferative responses in these cultures were measured after 3d.

### Dendritic cells

Dendritic cells belong to a family of antigen-presenting cells that are located in the T cell dependent area of lymphoid tissues. The dendritic cells are also found at the barrier zones where antigen entry into the human body may take place such as the skin, lung and gut. Recent evidence indicates a role for dermal dendritic cells in contact hypersensitivity reactions (Tse and Cooper, 1990, J Invest Derm 94:267), psoriasis (Nestle et al, 1994, J Clin Invest 94:202-209), lymphoproliferative disorders (Nestle et al, 1995, Dermatol 190:265-280). Dendritic cells have been studied for their potency for presentation of bacterial derived superantigens and soluble protein antigens to T cells (Nestle and Nickoloff, 1995, Adv Exp Med Biol 378:111-116).

### Results

Table 7 shows the 3H-Thymidine Incorporation in counts per minute (cpm) as a function of the numbers of stimulator cells for dendritic cells preincubated with oxytocin compared to dendritic cells preincubated with saline.

**Table 7**. 3H-Thymidine Incorporation in cpm as a function of the numbers of stimulator cells for dendritic cells preincubated with oxytocin compared to dendritic cells preincubated with saline.

| Numbers of stimulator cells | 3H-Thymidine Incorporation (cpm) | |
|---|---|---|
| | Saline | Oxytocin |
| 50 | 3,200 | 4,300 |
| 500 | 4,800 | 7,400 |
| 5,000 | 12,000 | 15,100 |

The results in Table 7 show that oxytocin increases the induction of dendritic cells TT specific T cell proliferation.

### Example 5 - Stimulation of dermal keratinocvtes

### Materials and methods

The procedure of Rheinwald JG and Green H. Serial cultivation of strains of human epidermal keratinocytes: the formation of keratinizing colonies from single cells. Cell 6:331-334 (1975), was used for cultivation of human keratinocytes. This procedure used serum-free keratinocyte medium supplemented with bovine pituitary extract and epidermal growth factor; primarily cultured keratinocytes at passages 3-5 were used. Keratinocytes were then challenged with oxytocin or saline. The cell media was then analysed for TGF-b1 using enzyme-linked immunosorbent assay (sandwich ELISA).

### Keratinocyte therapy

It is well known that TGF-b1 is one of the most effective growth factors stimulating the synthesis and deposition of various matrix proteins important in differentiation, morphogenesis and wound healing (Goldstein RH, Foliks CF, Pilch PF, Smith BD, and Fine A. Stimulation of collagen formation by insulin and insuline-like growth factor I in cultures of human lung fibroblasts. Endocrinol 124:964-970 (1989); Roberts AB and Sporn MB. In: Sporn MD and Roberts AB (eds). The Handbook of Experimental Pharmacology, Peptide Growth Factors and their Receptors. Springer-Verlag, Heidelberg, 1989, p. 419-472; Hill DJ, Logan A, McGarry M, De Sousa D: Control of protein and matrix-molecule synthesis in isolated bovine fetal growth-plate chondrocytes by the interactions of basic fibroblast growth factor, insulin-like growth factor-I and II, insulin and transforming growth factor-β1, J Endocrinol 133:363-373 (1992)). The process of cultivating keratinocytes in vivo to confluency for grafting onto patients with severe skin injuries, often thermal ones, has been as well established (Green H, Kehinde O, and Thomas J: Growth of cultured human epidermal cells into multiple epithelia suitable for grafting. Proc Nat Acad Sci (USA) 76:5665-5668, (1979)). Keratinocyte therapy, therefore, offers enormous potential for novel therapeutic approaches not only following thermal injury but also for other congenital and acquired disorders of the skin.

### Results

Table 8 shows the TGF-b 1 concentration in pg/mL in the cell media.

**Table 8. TGF-b1 concentration (pg/mL.) in cell media for keratinocytes challenged with oxytocin compared to keratinocytes challenged with saline.**

| | TGF-b1 concentration (pg/mL) |
|---|---|
| Keratinocytes + saline | 12 ± 4 |
| Keratinocytes + oxytocin | 34 ± 8 |

The results in Table 8 show that oxytocin increases the production of TGF-b1 from keratinocytes.

### Example 5 - Reduction of sulfur mustard induced cell death (apoptosis) in keratinocytes

### Materials and methods

Normal human epidermal keratinocytes were obtained as primary cultures and maintained in serum-free keratinocyte growth medium.

### The effects of sulfur mustard

Sulfur mustard induces blisters in the skin via calcium-calmodulin and caspase-dependent pathways. Because sulfur mustard is a strong alkylating agent, its ability to induce DNA damage via apurinic sites and endonucleolytic activation has been advanced as one possible pathway leading to vesication.

Similar to other agents that induce DNA strand breakage, sulfur mustard stimulate the nuclear protein poly ADP-ribose polymerase, which significantly reduces the concentrations of cellular nicotin-amide adenine dinucleotide and adenosine triphosphate, a mechanism proposed to induce cell death (apoptosis).

As it has been shown that sulfur mustard may induce apoptosis in keratinocytes, keratinocytes were administered oxytocin in parallel with challenge with sulfur mustard.

### Experimental design

The normal human epidermal keratinocytes were grown in tissue culture flasks to 70-80% confluency, then exposed to sulfur mustard diluted in keratinocyte growth medium, with or without oxytocin 1 mM, to final concentrations of 100 µM or 300 µM. Media was not changed for the duration of experiments. Cell viability was measured by the ability of cells to exclude trypan blue.

### Results

Apoptosis is characterized by the appearance of nucleosome-sized ladders. DNA isolated from normal human epidermal keratinocytes treated with 100 µM sulfur mustard was intact which was also the case with normal human epidermal keratinocytes treated with 300 µM sulfur mustard and 1 mM oxytocin. On the other hand normal human epidermal keratinocytes treated with 300 µM sulfur mustard showed nucleosome-sized ladders on visual inspection following analysis with agarose gel electrophoresis and ethidium bromide staining. Trypan blue exclusion at 24 h was 97% in control cells, 88% following treatment with 300 mU sulfur mustard and oxytocin and 57% in cells treated with 300 mU sulfur mustard only.

Taken together oxytocin reduces the toxicity of sulfur mustard indicating an anti-apoptotic effect.

### Example 6 - Effects of oxytoxin treatment on aged skin and barrier formation

### Materials and methods

The protocol of Hanley et al. J Invest Dermatol, 106:404-411 (1996); Hanley et al. J Clin Invest 97:2576-2584 (1996), was used.

### Previous studies

Because a protective barrier is essential for life, the development of the epidermis and stratum corneum must be completed prior to birth (Komuves et al, J Invest Dermatol 1998 Sep; 111(3):429-433). The epidermal permeability barrier is comprised of corneocytes embedded in a lipid enriched matrix. Recent studies, using an explant model of fetal rat skin development that closely parallels in utero development, have shown that hormones and other activators of members of the nuclear receptor family regulate permeability barrier ontogenesis by stimulating lipid metabolism and the formation of the extracellular lipid lamellae.The results of Komuves et al, demonstrate that several hormones and activators of nuclear hormone receptors regulate epidermal differentiation during fetal development, affecting key constituents of both keratohyalin granules and the cornified envelope. Thus, a variety of ligands/activators of nuclear receptors accelerate not only permeability barrier ontogenesis, but also the expression of structural proteins essential for stratum corneum formation.

Previous studies have shown that ontogeny of the epidermal permeability barrier and lung occur in parallel in the fetal rat. Gender also influences lung maturation, i.e., males exhibit delayed development. Sex steroid hormones exert opposite effects on lung maturation, with estrogens accelerating and androgens inhibiting. In a recent study, Hanley et al (1996) demonstrate that cutaneous barrier formation, measured as transepidermal water loss, is delayed in male fetal rats. Administration of estrogen to pregnant mothers accelerates fetal barrier development both morphologically and functionally. Competent barriers also form sooner in skin explants incubated in estrogen-supplemented media in vitro. In contrast, administration of dihydrotestosterone delays barrier formation both *in vivo* and *in vitro.* Finally, treatment of pregnant rats with the androgen antagonist flutamide eliminates the gender difference in barrier formation.

Aged epidermis displays altered drug permeability, increased susceptibility to irritant contact dermatitis, and often severe xerosis, suggesting compromise of the aged epidermal barrier. To delineate the functional, structural, and lipid biochemical basis of epidermal aging, Ghadially et al, J Clin Invest 1995 May; 95(5):2281-2290 compared barrier function in young (20-30 yr) vs aged (> 80 yr) human subjects, and in a murine model. Baseline transepidermal water loss in both aged humans and senescent mice was subnormal. However, the aged barrier was perturbed more readily with either acetone or tape stripping (18 ± 2 strippings vs 31 ± 5 strippings in aged vs young human subjects, respectively). Moreover, after either acetone treatment or tape stripping, the barrier recovered more slowly in aged than in young human subjects (50 and 80% recovery at 24 and 72 h, respectively, in young subjects vs 15% recovery at 24 h in aged subjects), followed by a further delay over the next 6 d. Similar differences in barrier recovery were seen in senescent vs young mice. Although the total lipid content was decreased in the stratum corneum of aged mice (approximately 30%), the distribution of ceramides (including ceramide 1), cholesterol, and free fatty acids was unchanged. Moreover, a normal complement of esterified, very long-chain fatty acids was present. Finally, stratum corneum lamellar bilayers displayed normal substructure and dimensions, but were focally decreased in number, with decreased secretion of lamellar body contents. Thus, assessment of barrier function in aged epidermis under basal conditions is misleading, since both barrier integrity and barrier repair are markedly abnormal. These functional changes can be attributed to a global deficiency in all key stratum corneum lipids, resulting in decreased lamellar bilayers in the stratum corneum interstices. This constellation of findings may explain the increased susceptibility of intrinsically aged skin to exogenous and environmental insults.

### Results

In investigating the effects of oxytocin (exchanged with estrogen in the research protocol) administration, our results indicate that oxytocin accelerates cutaneous barrier formation through an action directly on the skin. In another series of studies we set out to investigate if topical application of oxytocin could affect the total lipid content in aged skin. Daily repeated application of oxytocin for 21 days increased the total lipid content with 1.1-1.6 % in aged skin suggesting that topical application of oxytocin may have a protective effect towards exogenous and environmental insults.

## Claims

1. Use of a substance with oxytocin activity for the preparation of a pharmaceutical composition to improve cell generation in conditions chosen from Parkinson's disease, after transplantations, to recover proliferation of blood cells, improve damaged sight, hearing, olfactory sense, and taste and to relieve incontinence problems.

2. Use according to Claim 1, **characterized in that** the substance is selected from the group consisting of the following compounds: 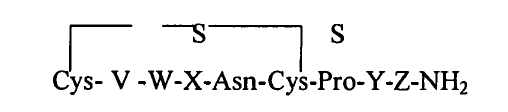 wherein
V is selected from the group consisting of Tyr and Phe
W is selected from the group consisting of Ile, Val, Hoph, Phe, and Cha,
X is selected from the group consisting of Gln, Ser, Thr, Cit, Arg, and Daba,
Y is selected from the group consisting of Ile, Leu, Thr, nothing, Val, Hos, Daba, and Cit,
Z is selected from the group consisting of Gly, nothing, and Ala.

3. Use according to Claim 1-2, **characterized in that** the substance is selected from the group consisting of the following compounds: oxytocin, mesotocin, isotocin, annetocin, SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, and SEQ ID NO: 9.

4. Use according to any of claims 1-3, **characterized** that the pharmaceutical composition further comprises substances that increase the release of oxytocin and/or the number or affinity of receptors, such as estrogen, or drugs having an α₂-agonistic effect, such as clonidine.

5. Use according to any of claims 1-4, **characterized** the effective concentration is 4-70% by weight, preferably 0.1-50% by weight.

6. Use according to any of claims 1-4, **characterized** the composition comprises an effective concentration of at least one substance with oxytocin activity.

7. A cosmetic method to improve cell generation by rejuvenating skin, counteract wrinkles, stimulate human dermal dendritic cells, stimulate dermal keratinocytes, reduce apoptosis, affect aged skin and barrier formation and assist in the growth of hair and nails, **characterized in that** a substance with oxytocin activity is administrated to an individual.

8. The method according to claims 7, **characterized in that** the substance is selected from the group consisting of the following compounds: 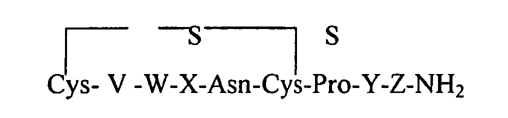 wherein
V is selected from the group consisting of Tyr and Phe
W is selected from the group consisting of Ile, Val, Hoph, Phe, and Cha,
X is selected from the group consisting of Gln, Ser, Thr, Cit, Arg, and Daba,
Y is selected from the group consisting of Ile, Leu, Thr, nothing, Val, Hos, Daba, and Cit,
Z is selected from the group consisting of Gly, nothing, and Ala.

9. The method according to any of claims 7-8, **characterized in that** the substance is selected from the group consisting of the following compounds: oxytocin, mesotocin, isotocin, annetocin, SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, and SEQ ID NO: 9.

10. The method according to any of claims 7-9, **characterized in that** a substance that increases the release of oxytocin and/or the number or affinity of receptors, such as estrogen, or drugs having an α₂-agonistic effect, such as clonidine is administrated to the individual in addition to the substance with oxytocin activity.

11. The method according to any of claims 7-10, **characterized in that** the effective concentration is 4-70% by weight, preferably 0.1-50% by weight.

12. The method according to any of claims 7-11, **characterized in that** the composition comprises an effective concentration of at least one substance with oxytocin activity.

## Patentansprüche

1. Verwendung einer Substanz mit Oxytocin-Aktivität für die Herstellung einer pharmazeutischen Zusammensetzung, um die Zellgeneration bei Zuständen, ausgewählt aus Parkinson-Erkrankung, nach Transplantationen zu verbessern, um die Proliferation von Blutzellen wieder herzustellen, um geschädigtes Sehvermögen, geschädigtes Hörvermögen, geschädigten Geruchssinn und geschädigten Geschmackssinn zu verbessern und Inkontinenzprobleme zu lindern.

2. Verwendung nach Anspruch 1, **dadurch gekennzeichnet, dass** die Substanz ausgewählt wird aus der Gruppe, bestehend aus den folgenden Verbindungen: 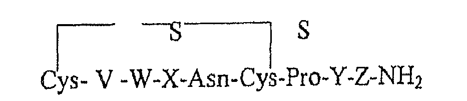 worin
V ausgewählt ist aus der Gruppe, bestehend aus Tyr und Phe,
W ausgewählt ist aus der Gruppe, bestehend aus Ile, Val, Hoph, Phe und Cha,
X ausgewählt ist aus der Gruppe, bestehend aus Gln, Ser, Thr, Cit, Arg und Daba,
Y ausgewählt ist aus der Gruppe, bestehend aus Ile, Leu, Thr, Nichts, Val, Hos, Daba und Cit,
Z ausgewählt ist aus der Gruppe, bestehend aus Gly, Nichts und Ala.

3. Verwendung nach den Ansprüchen 1 bis 2, **dadurch gekennzeichnet, dass** die Substanz ausgewählt ist aus der Gruppe, bestehend aus den folgenden Verbindungen: Oxytocin, Mesotocin, Isotocin, Annetocin, SEQ ID NO: 1; SEQ ID NO: 2, SEQ ID NO: 3, SEQ ID NO: 4, SEQ ID NO: 5, SEQ ID NO: 6, SEQ ID NO: 7, SEQ ID NO: 8 und SEQ ID NO: 9.

4. Verwendung nach einem der Ansprüche 1 bis 3, **dadurch gekennzeichnet, dass** die pharmazeutische Zusammensetzung außerdem Substanzen, die die Freisetzung von Oxytocin und/oder die Anzahl oder Affinität von Rezeptoren, z.B. Östrogen, oder Wirkstoffen mit α₂-Agonistenwirkung, z.B. Clonidin, erhöhen, umfasst.

5. Verwendung nach einem der Ansprüche 1 bis 4, **dadurch gekennzeichnet, dass** die wirksame Konzentration 4-70 Gew.-%, vorzugsweise 0,1-50 Gew.-%, ist.

6. Verwendung nach einem der Ansprüche 1 bis 4, **dadurch gekennzeichnet, dass** die Zusammensetzung eine wirksame Konzentration wenigstens einer Substanz mit Oxytocin-Aktivität umfasst.

7. Kosmetisches Verfahren zur Verbesserung der Zellgeneration unter Verjüngung der Haut, Entgegenwirken gegen Falten, Stimulieren der humanen dermalen dentritischen Zellen, Stimulieren der dermalen Keratinocyten, Verringern von Apoptose, Beeinflussen gealterter Haut und der Barrierebildung und Unterstützen des Wachstums von Haar und Nägeln, **dadurch gekennzeichnet, dass** eine Substanz mit Oxytocin-Aktivität an ein Individuum verabreicht wird.

8. Verfahren nach Anspruch 7, **dadurch gekennzeichnet, dass** die Substanz ausgewählt ist aus der Gruppe, bestehend aus den folgenden Verbindungen: 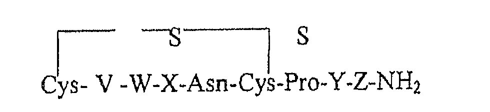 worin
V ausgewählt ist aus der Gruppe, bestehend aus Tyr und Phe,
W ausgewählt ist aus der Gruppe, bestehend aus Ile, Val, Hoph, Phe und Cha,
X ausgewählt ist aus der Gruppe, bestehend aus Gln, Ser, Thr, Cit, Arg und Daba,
Y ausgewählt ist aus der Gruppe, bestehend aus Ile, Leu, Thr, Nichts, Val, Hos, Daba und Cit,
Z ausgewählt ist aus der Gruppe, bestehend aus Gly, Nichts und Ala.

9. Verfahren nach einem der Ansprüche 7 bis 8, **dadurch gekennzeichnet, dass** die Substanz ausgewählt ist aus der Gruppe, bestehend aus den folgenden Verbindungen: Oxytocin, Mesotocin, Isotocin, Annetocin, SEQ ID NO: 1; SEQ ID NO: 2, SEQ ID NO: 3, SEQ ID NO: 4, SEQ ID NO: 5, SEQ ID NO: 6, SEQ ID NO: 7, SEQ ID NO: 8 und SEQ ID NO: 9.

10. Verfahren nach einem der Ansprüche 7 bis 9, **dadurch gekennzeichnet, dass** eine Substanz, die die Freisetzung von Oxytocin und/oder die Anzahl oder Affinität von Rezeptoren, z.B. Östrogen, oder von Wirkstoffen mit α₂-Agonistenwirkung, z.B. Clonidin, erhöht, dem Individuum zusätzlich zu der Substanz mit Oxytocin-Aktivität verabreicht wird.

11. Verfahren nach einem der Ansprüche 7 bis 10, **dadurch gekennzeichnet , dass** die wirksame Konzentration 4 bis 70 Gew.-%, vorzugsweise 0,1 bis 50 Gew.-%, ist.

12. Verfahren nach einem der Ansprüche 7 bis 11, **dadurch gekennzeichnet, dass** die Zusammensetzung eine wirksame Konzentration wenigstens einer Substanz mit Oxytocin-Aktivität umfasst.

## Revendications

1. Utilisation d'une substance ayant une activité ocytocique pour la préparation d'une composition pharmaceutique pour améliorer la génération cellulaire dans des conditions choisies parmi une maladie de Parkinson, après des transplantations, pour récupérer une prolifération de globules sanguins, améliorer la vue, l'ouïe, l'odorat et le goût endommagés et pour soulager des problèmes d'incontinence.

2. Utilisation selon la revendication 1, **caractérisée en ce que** la substance est sélectionnée dans le groupe constitué des composés suivants : 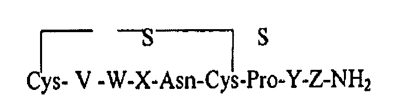 dans lequel
V est sélectionné dans le groupe constitué de Tyr et Phe
W est sélectionné dans le groupe constitué de Ile, Val, Hoph, Phe et Cha,
X est sélectionné dans le groupe constitué de Gln, Ser, Thr, Cit, Arg et Daba,
Y est sélectionné dans le groupe constitué de Ile, Leu, Thr, aucun, Val, Hos, Daba et Cit,
Z est sélectionné dans le groupe constitué de Gly, aucun, et Ala.

3. Utilisation selon les revendications 1 à 2, **caractérisée en ce que** la substance est sélectionnée dans le groupe constitué des composés suivants : ocytocine, mésotocine, isotocine, annétocine, SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8 et SEQ ID NO : 9

4. Utilisation selon l'une quelconque des revendications 1 à 3, **caractérisée en ce que** la composition pharmaceutique comprend en outre des substances qui augmentent la libération d'ocytocine et/ou le nombre ou l'affinité de récepteurs, comme l'oestrogène, ou des médicaments ayant un effet agoniste α₂, tel que la clonidine.

5. Utilisation selon l'une quelconque des revendications 1 à 4, **caractérisée en ce que** la concentration efficace est comprise entre 4 à 70 % en poids, de préférence 0,1 à 50 % en poids.

6. Utilisation selon l'une quelconque des revendications 1 à 4, **caractérisée en ce que** la composition comprend une concentration efficace d'au moins une substance avec une activité ocytocique.

7. Procédé cosmétique destiné à améliorer une génération cellulaire par un rajeunissement de la peau, contrer les rides, stimuler les cellules dendritiques dermiques humaines, stimuler les kératinocytes dermiques, réduire l'apoptose, avoir une incidence sur une peau âgée et la formation d'une barrière et aider à la croissance des cheveux et des ongles, **caractérisé en ce qu'**une substance ayant une activité ocytocique est administrée à un individu.

8. Procédé selon la revendication 7, **caractérisé en ce que** la substance est sélectionnée dans le groupe constitué des composés suivants : 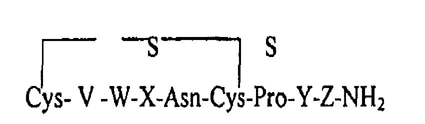 dans lequel
V est sélectionné dans le groupe constitué de Tyr et Phe
W est sélectionné dans le groupe constitué de Ile, Val, Hoph, Phe et Cha,
X est sélectionné dans le groupe constitué de Gln, Ser, Thr, Cit, Arg et Daba,
Y est sélectionné dans le groupe constitué de Ile, Leu, Thr, aucun, Val, Hos, Daba et Cit,
Z est sélectionné dans le groupe constitué de Gly, aucun, et Ala.

9. Procédé selon l'une quelconque des revendications 7 à 8, **caractérisé en ce que** la substance est sélectionnée dans le groupe constitué des composés suivants : ocytocine, mésotocine, isotocine, annétocine, SEQ ID NO : 1, SEQ ID NO : 2, SEQ ID NO : 3, SEQIDNO: 4, SEQIDNO: 5, SEQIDNO: 6, SEQIDNO: 7, SEQIDNO: 8 et SEQ ID NO : 9

10. Procédé selon l'une quelconque des revendications 7 à 9, **caractérisé en ce qu'**une substance qui augmente la libération de l'ocytocine et/ou le nombre ou l'affinité de récepteurs, comme l'oestrogène, ou des médicaments ayant un effet agoniste α₂, tel que la clonidine, est administrée à l'individu en plus de la substance ayant une activité ocytocique.

11. Procédé selon l'une quelconque des revendications 7 à 10, **caractérisé en ce que** la concentration efficace est comprise entre 4 à 70 % en poids, de préférence 0,1 à 50 % en poids.

12. Procédé selon l'une quelconque des revendications 7 à 11, **caractérisé en ce que** la composition comprend une concentration efficace d'au moins une substance ayant une activité ocytocique.


### SEQUENCE LISTING

<110> ENTRETECH MEDICAL AB
<120> A DRUG FOR CELL REGENERATION
<130> 53387
<140>
   <141>
< 160> 9
< 170> PatentIn Ver. 2.1
<210> 1
   <211> 7
   <212> PRT
   <213> Artificial Sequence
<220>
   <221> MOD_RES
   <222> (7)
   <223> AMIDATION
<220>
   <221> DISULFID
   <222> (1)..(6)
<220>
   <223> Description of Artificial Sequence:PEPTIDE
<400> 1
   Cys Tyr Ile Gln Asn Cys Pro
   1 5
<210> 2
   <211> 8
   <212> PRT
   <213> Artificial Sequence
<220>
   <221> MOD_RES
   <222> (8)
   <223> AMIDATION
<220>
   <221> DISULFID
   <222> (1)..(6)
<220>
   <223> Description of Artificial Sequence:PEPTIDE
<400> 2
   Cys Tyr Ile Gln Asn Cys Pro Leu
   1 5
<210> 3
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Description of Artificial Sequence:PEPTIDE
<400> 3
   Cys Tyr Val Thr Asn Cys Pro Leu Gly
   1 5
<210> 4
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Xaa(3)=Hoph
<220>
   <223> Description of Artificial Sequence:PEPTIDE
<400> 4
   Cys Tyr Xaa Thr Asn Cys Pro Val Gly
   1 5
<210> 5
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Xaa(4)=Cit
<220>
   <223> Description of Artificial Sequence:PEPTIDE
<400> 5
   Cys Tyr Phe Xaa Asn Cys Pro Leu Gly
   1 5
<210> 6
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<120>
   <223> Xaa(3)=Cha
<220>
   <223> Xaa(8)=Hos
<220>
   <223> Description of Artificial Sequence:PEPTIDE
<400> 6
   Cys Tyr Xaa Arg Asn Cys Pro Xaa Ala
   1 5
<210> 7
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Xaa(4)=Daba
<220>
   <223> Xaa(8)=Daba
<220>
   <223> Description of Artificial Sequence:PEPTIDE
<400> 7
   Cys Tyr Val Xaa Asn Cys Pro Xaa Ala
   1 5
<210> 8
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Xaa(3)=Hoph
<220>
   <223> Xaa(4)=Daba
<220>
   <223> Xaa(8)=Cit
<220>
   <223> Description of Artificial Sequence:PEPTIDE
<400> 8
   Cys Tyr Xaa Xaa Asn Cys Pro Xaa Ala
   1 5
<210> 9
   <211> 9
   <212> PRT
   <213> Artificial Sequence
<220>
   <221> MOD_RES
   <222> (9)
   <223> AMIDATION
<220>
   <221> DISULFID
   <222> (1)..(6)
<220>
   <223> Description of Artificial Sequence:PEPTIDE
<400> 9
   Cys Tyr Phe Arg Asn Cys Pro Val Ala
   1 5